# EUROPEAN PATENT APPLICATION

(11) **EP 1 803 459 A1**
(43) Date of publication of application: **04.07.2007**
(21) Application number: 05786003.3
(22) Date of filing: 26.09.2005
(51) Int. Cl.: A61K 33/24, A61K 47/04, A61P 35/00

(54) **ANTITUMOR COMPOSITION**

(30) Priority: 27.09.2004 JP 2004279106
(71) Applicant: KABUSHIKI KAISHA SANGI, Tokyo 104-0045 (JP)
(72) Inventor: SAKUMA, Shuji, c/o Kabushiki Kaisha Sangi, Tokyo 104-0054 (JP); ATSUMI, Kiminori, c/o Kabushiki Kaisha Sangi, Tokyo 104-0045 (JP); KIKUKAWA, Keiichiro, c/o Kabushiki Kaisha Sangi, Tokyo 104-0045 (JP)
(74) Representative: Swinkels, Bart Willem
(86) International application number: PCT/JP2005/017633
(87) International publication number: WO 2006/035717

(57) **Abstract**

Antitumor agents with high antitumor effect have much side-effect, and its activity decreases when the toxicity decreases. Thus, satisfactory antitumor effects are not always obtained. Therefore, development of an antitumor agent having a high antitumor effect, with less side-effect and low toxicity is awaited. The present invention is to provide an antitumor agent of fine particles, wherein platinum chloride, chloroplatinic acids and hydroxyapatite are blend. Platinum chloride and chloroplatinic acids are platinum tetrachloride, tetrachloroplatinate, hexachloroplatinate, disodium platinum tetrachloride, disodium platinum hexachloride, dipotassium platinum tetrachloride, or dipotassium platinum hexachloride. The maximum particle diameter of hydroxyapatite is 1 µm or less.

## Description

### Technical Field

The present invention relates to an antitumor composition containing platinum chloride, chloroplatinic acids as active components, with less side-effect, and showing an excellent anticancer effect, especially for lung cancer.

### Background Art

For platinum, cisplatin (CDDP), carboplatin, ibroplatin or the like are already used as platinum complex. However, cisplatin which has a high antitumor effect has also intensive side-effects, which are excruciating for patients and its use is often limited. Moreover, as its water solubility is low, it can be supplied only at a low concentration. Further, carboplatin and ibroplatin which have fewer side effects compared to cisplatin, show lower activity when toxicity becomes low. Thus, it cannot be said that satisfactory antitumor effects are obtained.
Therefore, development of an antitumor agent having a high antitumor effect, with less side-effect and low toxicity is awaited.

On the other hand, hydroxyapatite is a dominant inorganic component of bones or teeth, shown by the stoichiometry composition Ca₁₀(PO₄)₆(OH)₂, having a good in vivo affinity, and an activity to adsorb proteins and the like. Therefore it has been commercialized as artificial bones, bone-grafting materials, tooth polishing agents and the like, and are variously designed and disclosed as a supplementary component for drugs.

As antitumor agents, the followings are disclosed: a method for suppressing tumor growth by injecting hydroxyapatite to which an anticancer drug is adsorbed into artery leading to a tumor site, and by cutting nutritional supply to the tumor as a microemblus (Japanese Laid-Open Patent Application No. 1-51266); a method for releasing drugs in a sustained manner by implanting hydroxyapatite embedded with an antitumor agent (Japanese Laid-Open Patent Application No. 2-200628); a method for activating a drug, by adding the drug to a calcium phosphate microcrystal, and promoting or delaying drug effects, or selectively adsorbing on various cells including cancer cells (Japanese Laid-Open Patent Application No. 5-255095); a method for using platinum-supported hydroxyapatite as an antitumor agent (Japanese Laid-Open Patent Application No. 4-112832); a method comprising the steps of embedding hydroxyapatite, to which an antitumor agent is adsorbed, to a tumor site, heating, and using the same for a thermochemotherapy. ("Cancer and Chemotherapy" 19(10):1644-1647, 1992); a method for sustained-releasing a carboplatin, by using a porous hydroxyapatite which average particle diameter is 36.1 µm and surface area is 2.5 m²/g as an sustained-release agent, preparing and adjusting a sustained-release carboplatin ("Cancer and Chemotherapy" 28(12): 1791-1793, 1999).

Patent Reference 1: Japanese Laid-Open Patent Application No. 1-51266
Patent Reference 2: Japanese Laid-Open Patent Application No. 2-200628
Patent Reference 3: Japanese Laid-Open Patent Application No. 5-255095
Patent Reference 4: Japanese Laid-Open Patent Application No. 4-112832
Patent Reference 5: Japanese Laid-Open Patent Application No. 4-112833
Non-Patent Reference 1: "Cancer and Chemotherapy", 19(10), p. 1644-1647, 1992
Non-Patent Reference 2: "Cancer and Chemotherapy", 26(12), p. 1791-1793, 1999

### Disclosure of the Invention

### Object to be solved by the Invention

The present invention is intended to provide an antitumor agent having an excellent antitumor effect, with low toxicity.

### Means to solve the Object

The present invention is characterized by that platinum chloride and platinum chloride acids are supported by hydroxyapatite microcrystal, as an antitumor agent with low toxicity, and having a high antitumor activity.
As an antitumor agent using platinum chloride acid as a raw material, a method for using a platinum-supported apatite chloride as an antitumor agent (Japanese Laid-Open Patent Application No. 4-112833) is disclosed, beside the above method for using a platinum-supported hydroxyapatite as an antitumor agent. In any of experiments with mice, the surviving rate of mice was higher compared to when cisplatin is used, while it cannot be said that the antitumor effect is significantly excellent. In the present invention, it was found that by compounding a platinum chloride which has a high toxicity and cannot be used as an antitumor agent and/or platinum chloride acids, with a hydroxyapatite, the toxicity was significantly reduced while retaining a high antitumor effect of platinum chloride.

As platinum chloride and chloroplatinic acids used in the present invention, platinum chlorides which are commonly marketed such as platinum tetrachloride PtCl₄, tetrachloroplatinate H₂PtCl₄, hexachloroplatinate H₂PtCl₆, disodium platinum tetrachloride Na₂PtCl₄, disodium platinum hexachloride Na₂PtCl₆, dipotassium platinum tetrachloride K₂PtCl₄, dipotassium platinum hexachloride K₂PtCl₆, or hydrates thereof, such as PtCl₄/5H₂O, H₂PtCl₆/6H₂O, Na₂PtCl₄/4.5H₂O, Na₂PtCl₆/4H₂O can be used.

Moreover, a hydroxyapatite used in the present invention is usually shown by the stoichiometry composition Ca₁₀(PO₄)₆(OH)₂. However, even when it is non-stoichiometric, for example, Ca/P molar ratio being 1.67 or less, it shows a property of hydroxyapatite, and can have an apatite construction. In the present invention, any hydroxyapatite having stoichiometry composition or non-stoichiometry composition can be used, and those which Ca/P molar ratio is 1.4 - 1.8 can be used.

Control of Ca/P molar ratio of hydroxyapatite is performed by controlling the mixing ratio of salt, raw material, and the synthetic conditions. For example, in a wet-type synthetic method of hydroxyapatite, by adjusting a water solution to be basic with aqueous ammonia or the like, Ca/P molar ratio increases, and when adjusting a water solution to neutral to weak acidic with dilute acid, Ca/P molar ratio can be lowered.
As an above-mentioned hydroxyapatite used in the present invention, either of crystalline or less-crystalline ones can be used, while less-crystalline or noncrystalline ones are preferred.

The term "less-crystalline" herein used relates to a crystalline powder wherein X-ray diffraction peak is broader compared to that of a highly crystalline powder.
The term "noncrystalline" relates to a powder consisting of fine particles wherein X-ray diffraction pattern shows a broad halo and a diffraction pattern showing crystalline characteristics cannot be obtained. Hereinafter, less-crystalline hydroxyapatite and noncrystalline hydroxyapatite will be referred to as "less-crystalline apatite", and "noncrystalline apatite", respectively.
As a less-crystalline apatite or noncrystalline apatite used in the present invention, an apatite synthesized by a wet-type method mentioned in the above, lyophilized or dried at a temperature of 100°C or less, or burned at a temperature of approximately 300°C or less, is used.
A less-crystalline apatite or noncrystalline apatite is consisted of particles with smaller diameter compared to that of high-crystalline hydroxyapatite (herein after referred to as "crystalline apatite").

As a hydroxyapatite particle used in the present invention, a particle which maximum particle diameter is 1.0 µm or less is preferred from the point of view of intravascular administration, and a particle which maximum particle diameter is 0.1 µm or less is more preferred from the point of view of antitumor effect. As a lower limit of the particle diameter, as calcium elutes when the hydroxyapatite particle is small, an average particle diameter of 0.02 µm or more is preferred.
A method for adjusting the size of hydroxyapatite particles can be performed by grinding. With a hydroxyapatite with a maximum particle diameter of 1.0 µm or less, preferably with a maximum particle diameter of 0.1 µm or less, an antitumor agent can be obtained by grinding the hydroxyapatite so that the lower limit of average particle diameter is 0.02 µm, and mixing with platinum chlorides. However, it is more preferred to grind after mixing platinum chlorides and hydroxyapatite, and to adjust to the above particle diameter, from the point of view of the effect of lowering the toxicity of platinum chlorides.

The blending level of platinum chloride and chloroplatinic acids with hydroxyapatite is preferably 0.1 to 100%, and a level of 1 to 30% is preferred in order to obtain a more sufficient toxicity decreasing effect, or from the point of view of dosage of antitumor agent.
In the following, the examples of the present invention will be explained. The following examples describe examples used for measuring LD50 as a toxicity test, and the scope of the present invention will not be limited by these.

### Brief Description of Drawings

[Fig. 1] It is a microgram showing a culture cell image to which a platinum added-CDDP dispersion liquid contained in IC50 of CDDP has been added to a resting stage fibroblast (10 fold).
[Fig. 2] It is a microgram showing a culture cell image to which a platinum added-PtCl₄/HAP dispersion liquid contained in IC50 of CDDP has been added to a resting stage fibroblast (10 fold).
[Fig. 3] It is a microgram showing a culture cell image to which a platinum added-PtCl₄ dispersion liquid contained in IC50 of CDDP has been added to a resting stage fibroblast (10 fold).
[Fig. 4] It is a microgram showing a culture cell image to which a platinum added-HPA dispersion liquid contained in IC50 of CDDP has been added to resting stage fibroblast (10 fold).

### Best Mode of Carrying Out the Invention

In the following, the present invention will be described more in detail by referring to the Examples. (Platinum tetrachloride/hydroxyapatite-antitumor agent)

### [Example 1] Raw material to prepare a 10% platinum tetrachloride/90% hydroxyapatite-antitumor agent

1. Hydroxyapatite (HAP)
2. Platinum tetrachloride (PtCl₄)
3. Sodium hydroxide: pH adjuster
4. Citric acid: dispersant

1 g of platinum tetrachloride was weighed, to be added and stirred in a distilled water previously degassed. Then, sodium hydroxide was added to adjust pH to neutral region (pH 6.8 to 7.4). 9 g of hydroxyapatite was weighed and added to this solution, stirred in vacuo. Distilled water was added in the solution to make a total volume of 500 ml, and the resultant was agitated for approximately 1 month, at a constant temperature of 50 to 60°C in dark, in a sealed container.
When agitation was stopped, the resultant was concentrated with an evaporator, lyophilized to obtain a powder.
Distilled water was added to the powder in order to adjust the powder concentration to be approximately 2%. Further, a dispersant wherein citric acid was adjusted to pH of approximately 7 . 0 with sodium hydroxide was added therein so that a dispersant concentration becomes 20 mM/L.
This solution was ground with a DYNO-MILL (Willy A. Bachofen AB, Switzerland), a wet-type dispersion/grinding machine, by using a circulating pump for cooling, so that the grinding temperature becomes 3 to 5°C. A solution of particles of 0.05 to 0.1 µm, which average particle diameter is approximately 0.07 µm was obtained.
The solution was filtered with a 0.22 µm sterilizing filter for sterilization, to obtain a platinum tetrachloride/hydroxyapatite-antitumor agent (PtCl₄/HAP). The obtained antitumor agent was subjected to X-ray analysis and E-MAX for structural analysis and analysis of distribution of each element.

Tube voltage and tube current of the X-ray diffraction device were 45 KV and 300 mA, respectively. Measurement was performed by using a CuKα line threaded with a nickel filter, under conditions of divergency slit 1.0 deg, scatter slit 1.0 deg, and receiving slit 0.3 mm. Measurement was 5,000 to 7,000 deg, sampling distance 0.0500 deg, and scanning speed 4,000 deg/min.
The obtained diffraction result was analyzed by using Match Maker (Mac Science) attached to the device.
Further, E-MAX analysis was performed with energy dispersive X-ray analysis, attached to the scanning electron microscope.
S-2380N of HITACHI Ltd. was used as a scanning electron microscope, and EMAX-5770W, energy dispersive X-ray analyzer of HORIBA Ltd. was used for analysis of distribution of each ion. Observation of platinum apatite by a scanning electron microscope was performed under conditions of accelerating voltage of 20 kV, and working distance of 25 mm. For element analysis by energy dispersive X-ray analyzer under scanning electron microscope, the conditions were 600 sec for integrative time for detected elements, and 20% for dead time (DT). Moreover, as mapping conditions of each detected element, measurement time was 20 min/frame, accumulated time 227 times, and pixel count 512.
To subject an antitumor agent to scanning electron microscope, a sample was set on a stage and coated with carbon.

As a result of X-ray diffraction, no phase change was observed for platinum tetrachloride and hydroxyapatite in the antitumor agent and there was no structural change.
Further, as a result of E-MAX analysis, calcium and phosphorus, main elements constituting hydroxyapatite, were observed throughout particles of an antitumor agent. It was further observed that platinum and chlorine were distributed only on and throughout hydroxyapatite particles. Therefore, it was confirmed to be a compound wherein all platinum tetrachlorides are supported by hydroxyapatite.
With a similar preparation method, by changing only the ratio of platinum tetrachloride and hydroxyapatite, antitumor agents wherein the ratio of platinum tetrachloride to hydroxyapatite is 0.1%, 1%, 5%, 20%, 50%, and 100% were obtained.
Further, antitumor agents with a maximum particle size of 1 µm and an average particle diameter of approximately 0.4 µm, which ratio of platinum tetrachloride to hydroxyapatite is 0.1%, 1%, 5%, 10%, 20%, 50%, and 100% were obtained.
Those antitumor agents were subjected to X-ray analysis and E-MAX for structural diffraction and analysis of distribution of each element in a similar manner as in the above, and similar results were obtained.

### (hexachloroplatinate/hydroxyapatite-antitumor agent)

### [Example 2]

Raw material to prepare a 10% hexachloroplatinate/90% hydroxyapatite-antitumor agent
1. Hydroxyapatite (HAP)
2. Hexachloroplatinate (H₂PtCl₆)
3. Sodium hydroxide: pH adjuster
4. Sodium citrate: dispersant

1.005 g of hexachloroplatinate was weighed, to be added and stirred in a distilled water previously degassed. Then, sodium hydroxide was added to adjust pH to neutral region (pH 6.8 to 7.4). 9 g of hydroxyapatite was weighed and added to this solution, stirred in vacuo. Distilled water was added to the solution to make a total volume of 200 ml, and the resultant was agitated for approximately 2 month, in dark, in a sealed container.
When agitation was stopped, the resultant was lyophilized to obtain a powder of hexachloroplatinate and hydroxyapatite.
Distilled water was added to the powder, in order to adjust the powder concentration to approximately 1%. Further, sodium citrate was added so that the sodium citrate concentration becomes 10 mM/l as a dispersant.
This solution was ground with a DYNO-MILL (Willy A. Bachogen AB, Switzerland), a wet-type dispersion/grinding machine, by using a circulating pump for cooling, so that the grinding temperature becomes 3 to 5°C. A solution of particles of 0.02 to 0.07 µm, which average particle diameter is approximately 0.05 µm was obtained.
The solution was filtered with a 0.22 µm sterilizing filter for sterilization, to obtain a hexachloroplatinate/hydroxyapatite-antitumor agent (H₂PtCl₆/HAP).

As a result of structural analysis, the antitumor agent was a compound wherein all of hexachloroplatinates are supported by hydroxyapatite.
With a similar preparation method, by changing only the ratio of hexachloroplatinate and hydroxyapatite, antitumor agents wherein the ratio of hexachloroplatinate to hydroxyapatite is 0.1%, 1%, 5%, 20%, 50%, and 100% were obtained.
Further, antitumor agents with a maximum particle size of 1 µm and an average particle diameter of approximately 0.3 µm, which ratio of platinum hexachloride to hydroxyapatite is 0.1%, 1%, 5%, 10%, 20%, 50%, and 100% were obtained.
These antitumor agents were also subjected to X-ray analysis and E-MAX for structural diffraction and analysis of distribution of each element, in a similar manner as Example 1. As a result, they were mixtures wherein all of hexachloroplatinates are supported by hydroxyapatite.

### (disodium platinum hexachloride/hydroxyapatite-antitumor agent)

### [Example 3] Preparation of a 10% disodium platinum hexachloride/90% hydroxyapatite-antitumor agent Raw material

1. Hydroxyapatite (HAP)
2. disodium platinum hexachloride (NA₂PtCl₆)
3. Sodium hydroxide: pH adjuster
4. Citric acid: dispersant

1 g of disodium platinum hexachloride was weighed, to be added and stirred in a distilled water previously degassed. Then, pH was adjusted to neutral region (pH 6.8 to 7.4) by adding sodium hydroxide. 9 g of hydroxyapatite was weighed and added to this solution, stirred in vacuo. Distilled water was added in the solution to make a total volume of 500 ml, and the resultant was agitated for approximately 1 month, at a constant temperature of 50 to 60°C in dark, in a sealed container.
When agitation was stopped, the resultant was concentrated with an evaporator, and lyophilized to obtain a powder.
Distilled water was added to the powder, in order to adjust the powder concentration to approximately 2%. Further, a dispersant wherein citric acid was adjusted so that pH becomes approximately 7.0 with sodium hydroxide was added so that a dispersant concentration becomes 20 mM/l.
This solution was ground with a DYNO-MILL (Willy A. Bachogen AB, Switzerland), a wet-type dispersion/grinding machine, by using a circulating pump for cooling, so that the grinding temperature becomes 3 to 5°C. A solution of particles of 0.05 to 0.1 µm, which average particle diameter is approximately 0.07 µm was obtained.
The solution was filtered with a 0.22 µm sterilizing filter for sterilization, to obtain a disodium platinum hexachloride/hydroxyapatite-antitumor agent (Na₂PtCl₆/HAP).

As a result of structural analysis, the antitumor agent was a mixture wherein all of disodium platinum hexachlorides were supported by hydroxyapatite.
With a similar preparation method, by changing only the ratio of disodium platinum hexachloride and hydroxyapatite, antitumor agents wherein the ratio of disodium platinum hexachloride to hydroxyapatite is 0.1%, 1%, 5%, 20%, 50%, and 100% were obtained.
Further, antitumor agents with a maximum particle size of 1 µm and an average particle diameter of approximately 0.3 µm, which ratio of disodium platinum hexachloride is 0.1%, 1%, 5%, 10%, 20%, 50%, and 100% were obtained.
These antitumor agents were also subjected to X-ray analysis and E-MAX for structural diffraction and analysis of distribution of each element, in a similar manner as Example 1. As a result, they were mixtures wherein all of disodium platinum hexachloride are supported by hydroxyapatite.

### (dipotassium platinum tetrachloride/hydroxyapatite-antitumor agent)

### [Example 4] Preparation of a 10% disodium platinum tetrachloride/90% hydroxyapatite-antitumor agent Raw material

1. Hydroxyapatite (HAP)
2. Dipotassium platinum tetrachloride (K₂PtCl₄)
3. Sodium hydroxide: pH adjuster
4. Sodium citrate: dispersant

1 g of dipotassium platinum tetrachloride was weighed, to be added and stirred in a distilled water previously degassed. Then, sodium hydroxide was added to adjust pH to neutral region (pH 6.8 to 7.4). 9 g of hydroxyapatite was weighed and added to this solution, stirred in vacuo. Distilled water was added to the solution to make a total volume of 500 ml, and the resultant was agitated for approximately 1 month, at a constant temperature of 50 to 60°C in dark, in a sealed container.
When agitation was stopped, the resultant was concentrated with an evaporator, lyophilized to obtain a powder.
Distilled water was added to the powder, in order to adjust the powder concentration to approximately 2%. Further, a dispersant wherein citric acid was adjusted so that pH becomes approximately 7.0 with sodium hydroxide was added so that a dispersant concentration becomes 20 mM/L.
This solution was ground with a DYNO-MILL (Willy A. Bachogen AB, Switzerland), a wet-type dispersion/grinding machine, by using a circulating pump for cooling, so that the grinding temperature becomes 3 to 5°C. A solution of particles of 0.05 to 0.1 µm, which average particle diameter is approximately 0.07 µm was obtained.
The solution was filtered with a 0.22 µm sterilizing filter for sterilization, to obtain dipotassium platinum tetrachloride/hydroxyapatite-antitumor agent (K₂PtCl₄/HAP).

As a result of structural analysis, the antitumor agent was a mixture wherein all of dipotassium platinum tetrachlorides were supported by hydroxyapatite.
With a similar preparation method, by changing only the ratio of dipotassium platinum tetrachloride and hydroxyapatite, antitumor agents wherein the ratio of dipotassium platinum tetrachloride to hydroxyapatite is 0.1%, 1%, 5%, 20%, 50%, and 100% were obtained.
Further, antitumor agents with a maximum particle size of 1 µm and an average particle diameter of approximately 0.3 µm, which ratio of dipotassium platinum tetrachloride is 0.1%, 1%, 5%, 10%, 20%, 50%, and 100% were obtained.
These antitumor agents were also subjected to X-ray analysis and E-MAX for structural diffraction and analysis of distribution of each element, in a similar manner as Example 1. As a result, they were mixtures wherein all of dipotassium platinum tetrachlorides platinum are supported by hydroxyapatite.

Further, by using tetrachloroplatinate, disodium platinum tetrachloride, dipotassium platinum tetrachloride and hydroxyapatite, the same operations as above were performed, and tetrachloroplatinate /hydroxyapatite (H₂PtCl₄/HAP)-antitumor agent, disodium platinum tetrachloride/hydroxyapatite (Na₂PtCl₄/HAP)-antitumor agent, and dipotassium platinum hexachloride/hydroxyapatite (K₂PtCl₆/HAP)-antitumor agent were obtained.
These antitumor agents were also subjected to X-ray analysis and E-MAX for structural diffraction and analysis of distribution of each element, in a similar manner as Example 1. As a result, they were mixtures wherein all of each platinic compound are supported by hydroxyapatite.

### [Example 5]

### Effect of platinum tetrachloride/hydroxyapatite (PtCl₄/HAP) and hexachloroplatinate/hydroxyapatite (H₂PtCl₆/HAP) against human cancer cells (MTT assay)

According to "in vitro toxicity test using MTT", which is an ordinary test method, MTT assay was performed for PtCl₄/HAP (particle diameter 0.1 µm or less), and H₂PtCl₆/HAP (particle diameter 0.1 µm or less) by using 19 types of human cancer cells. PtCl₄/HAP and H₂PtCl₆/HAP were used by adjusting the final dispersion concentration to 1.0% in a dispersion liquid each containing 10% platinum as a theoretical value. For comparison, MTT assay was performed for PtCl₄ and CDDP. PtCl₄ was dissolved at an adequate dose, and then used after serial dilution with PBS (-). For CDDP, injection liquid from Malco was used.
IC50 by MTT assay are shown in Table 1. Further, numeric values wherein IC50 are converted to platinum concentration are shown in Table 2.
For tetrachloroplatinate/hydroxyapatite (H₂PtCl₄/HAP)-antitumor agents, hexachloroplatinate/hydroxyapatite (H₂PtCl₆/HAP) -antitumor agents, disodium platinum tetrachloride/hydroxyapatite (Na₂PtCl₄/HAP) -antitumor agents, disodium platinum hexachloride/hydroxyapatite (Na₂PtCl₆/HAP)-antitumor agents, dipotassium platinum tetrachloride/hydroxyapatite(K₂PtCl₄/HAP)-antitumor agents and dipotassium platinum hexachloride/hydroxyapatite(K₂PtCl₆/HAP)-antitumor agents, with particle diameter of 0.1 µm or less, and of 1.0 µm or less, and tetrachloroplatinate (H₂PtCl₄), hexachloroplatinate (H₂PtCl₆), disodium platinum tetrachloride (Na₂PtCl₄), disodium platinum hexachloride (Na₂PtCl₆), dipotassium platinum tetrachloride (K₂PtCl₄), and dipotassium platinum hexachloride (K₂PtCl₆) for comparison, IC 50 using human lung cancer cell Lu65 are shown in Table 3. Further, numeral values wherein IC50 are converted to platinum concentration are shown in Table 4.

**[Table 1]**

| IC50 (ppm) of human cancer cells by MTT assay | | | | | |
|---|---|---|---|---|---|
| | | PtCl₄/HAP | H₂PtCl₆/HAP | PtCl₄ | CDDP |
| Cervical cancer | HeLa | 47.0 | 49.4 | 7.0 | 2.1 |
| | SiHa | 51.9 | 55.1 | 8.7 | 15.1 |
| Fibrosarcoma | HT1080 | 61.3 | 67.5 | 9.6 | 25.5 |
| Squamous cell cancer | A431 | 113.6 | 114.9 | 9.5 | 5.7 |
| oral cancer | HSC3 | 58.3 | 63.0 | 9.6 | 2.9 |
| | HSC4 | 31.2 | 36.7 | 5.7 | 4.5 |
| | KB | 68.6 | 72.3 | 15.0 | 6.3 |
| esophageal cancer | YES1 | 177.7 | 169.2 | 19.2 | 8.2 |
| | YES2 | 211.0 | 193.0 | 35.8 | 1.7 |
| | YES3 | 66.8 | 80.8 | 19.2 | 1.2 |
| | YES4 | 33.6 | 56.2 | 14.6 | 1.3 |
| | YES5 | 102.3 | 105.4 | 20.1 | 10.0 |
| | YES6 | 119.7 | 151.1 | 11.9 | 0.8 |
| gastric cancer | AZ521 | 126.8 | 143.6 | 14.6 | 1.0 |
| | Kato-III | 23.7 | 30.7 | 5.8 | 2.7 |
| | NUGC-4 | 44.4 | 48.7 | 7.7 | 15.6 |
| colon cancer | SW837 | 210.6 | 210.8 | 32.9 | 40.2 |
| | LoVo | 26.8 | 32.9 | 6.6 | 15.8 |
| liver cancer | HepG2 | 69.3 | 85.0 | 6.2 | 5.0 |
| lung cancer | Lu65 | 1.9 | 3.8 | 0.9 | 2.7 |
| | Lu99 | 4.1 | 3.4 | 1.1 | 0.8 |

**[Table 2]**

| Platinum content (ppm) calculated from IC50 of human cancer cells by MTT assay | | | | | |
|---|---|---|---|---|---|
| | | PtCl₄/HAP | H₂PtCl₆/HAP | PtCl₄ | CDDP |
| Cervical cancer | HeLa | 4.7 | 4.9 | 4.0 | 1.4 |
| | SiHa | 5.2 | 5.5 | 5.0 | 9.8 |
| Fibrosarcoma | HT1080 | 6.1 | 6.8 | 5.6 | 16.6 |
| Squamous cell cancer | A431 | 11.4 | 11.5 | 5.5 | 3.7 |
| oral cancer | HSC3 | 5.8 | 6.3 | 5.5 | 1.9 |
| | HSC4 | 3.1 | 36.7 | 3.3 | 2.9 |
| | KB | 6.9 | 72.3 | 8.7 | 4.1 |
| esophageal cancer | YES1 | 17.8 | 16.9 | 11.1 | 5.4 |
| | YES2 | 21.1 | 19.3 | 20.7 | 1.1 |
| | YES3 | 6.7 | 8.1 | 11.1 | 0.8 |
| | YES4 | 3.4 | 5.6 | 8.5 | 0.8 |
| | YES5 | 10.2 | 10.5 | 11.6 | 6.5 |
| | YES6 | 12.0 | 15.1 | 6.9 | 0.5 |
| gastric cancer | AZ521 | 12.7 | 14.4 | 8.4 | 0.7 |
| | KATO-III | 2.4 | 3.1 | 3.4 | 1.8 |
| | NUGC-4 | 4.4 | 4.9 | 4.5 | 10.1 |
| colon cancer | SW837 | 21.1 | 21.1 | 19.1 | 26.1 |
| | LoVo | 2.7 | 3.3 | 3.8 | 10.3 |
| liver cancer | HepG2 | 6.9 | 8.5 | 6.2 | 5.0 |
| lung cancer | Lu65 | 0.2 | 0.4 | 0.9 | 2.7 |
| | Lu99 | 0.4 | 0.3 | 1.1 | 0.8 |

**[Table 3]**

| IC50 (ppm) of human lung cancer cells Lu65, Lu99 by MTT assay | | | | |
|---|---|---|---|---|
| Human lung cancer cell Lu65 | | | | |
| sample | 0.1 µm or less (ppm) | 1.0 µm or less (ppm) | sample | (ppm) |
| H₂PtCl₄/HAP | 3.2 | 14.1 | H₂PtCl₄ | 8.8 |
| H₂PtCl₆/HAP | 3.8 | 7.2 | H₂PtCl₆ | 11.1 |
| Na₂PtCl₄/HAP | 6.4 | 18.4 | Na₂PtCl₄ | 8.8 |
| Na₂PtCl₆/HAP | 6.9 | 20.0 | Na₂PtCl₆ | 10.5 |
| K₂PtCl₄/HAP | 6.6 | 30.9 | K₂PtCl₄ | 58.2 |
| K₂PtCl₆/HAP | 9.1 | 48.5 | K₂PtCl₆ | 41.7 |
| | | | | |

| Human lung cancer cell Lu99 | | | | |
|---|---|---|---|---|
| sample | 0.1 µm or less (ppm) | 1.0 µm or less (ppm) | sample | (ppm) |
| H₂PtCl₄/HAP | 1.8 | 5.2 | H₂PtCl₄ | 9.1 |
| H₂PtCl₆/HAP | 3.4 | 5.0 | H₂PtCl₆ | 7.2 |
| Na₂PtCl₄/HAP | 11.1 | 21.9 | Na₂PtCl₄ | 9.2 |
| Na₂PtCl₆/HAP | 2.7 | 6.4 | Na₂PtCl₆ | 4.7 |
| K₂PtCl₄/HAP | 4.3 | 12.5 | K₂PtCl₄ | 12.1 |
| K₂PtCl₆/HAP | 1.6 | 8.1 | K₂PtCl₆ | 4.1 |

**[Table 4]**

| Platinum content (ppm) calculated from IC50 of human lung cancer cell Lu65, Lu99 by MTT assay | | | | |
|---|---|---|---|---|
| Human lung cancer cell Lu65 | | | | |
| sample | 0.1 µm or less (ppm) | 1.0 µm or less (ppm) | sample | (ppm) |
| H₂PtCl₄/HAP | 0.3 | 1.4 | H₂PtCl₄ | 5.1 |
| H₂PtCl₆/HAP | 0.4 | 0.7 | H₂PtCl₆ | 5.3 |
| Na₂PtCl₄/HAP | 0.6 | 1.8 | Na₂PtCl₄ | 4.5 |
| Na₂PtCl₆/HAP | 0.7 | 2.0 | Na₂PtCl₆ | 4.5 |
| K₂PtCl₄/HAP | 0.7 | 3.1 | K₂PtCl₄ | 27.4 |
| K₂PtCl₆/HAP | 0.9 | 4.9 | K₂PtCl₆ | 16.7 |
| | | | | |

| Human lung cancer cell Lu99 | | | | |
|---|---|---|---|---|
| sample | 0.1 µm or less (ppm) | 1.0 µm or less (ppm) | sample | (ppm) |
| H₂PtCl₄/HAP | 0.2 | 0.5 | H₂PtCl₄ | 4.3 |
| H₂PtCl₆/HAP | 0.3 | 0.5 | H₂PtCl₆ | 3.4 |
| Na₂PtCl₄/HAP | 1.1 | 2.2 | Na₂PtCl₄ | 4.7 |
| Na₂PtCl₆/HAP | 0.3 | 0.6 | Na₂PtCl₆ | 2.0 |
| K₂PtCl₄/HAP | 0.4 | 1.3 | K₂PtCl₄ | 5.7 |
| K₂PtCl₆/HAP | 0.2 | 0.8 | K₂PtCl₆ | 1.6 |

### (Toxicity test and cell-killing effect for normal cells)

A male mature rat was deeply anesthetized with ether, and disinfected by wiping carefully its tail with alcohol. The tail end part was cut by several centimeters, and the skin was removed from coccyx. Tendon was attached to the coccyx, and by culturing the same, fibroblasts can be recovered. Coccyx part was further cut finely with a sterilized pair of scissors, and adhered on a sterilized plastic plate with the cut surface down. After resting the plate for a while, DMEM supplemented with 10% FBS was added thereto. In approximately one week, fibroblasts germinated from the coccyx part, and proliferated in the plate. The fibroblasts collected from rat tail end were used for the following experiment.

### [Example 6]

### Toxicity test of platinum tetrachloride/hydroxyapatite (PtCl₄/HAP) and hexachloroplatinate (H₂PtCl₆/HAP) during proliferation stage

According to "in vitro toxicity test method using MTT" which is an ordinary test method, MTT assay was performed for PtCl₄/HAP (particle diameter 0.1 µm or less) and H₂PtCl₆/HAP (particle diameter 0.1 µm or less). PtCl₄/HAP and H₂PtCl₆/HAP were used by adjusting the final dispersion concentration to 1.0%, with a dispersion liquid each containing 10% platinum as a theoretical value. For comparison, MTT assay was performed for PtCl₄ and CDDP. PtCl₄ was dissolved at an adequate dose, and then used after serial dilution with PBS (-). For CDDP, injection liquid from Malco was used.

Fibroblasts during proliferation stage on the plate were peeled off with trypsin-EDTA, adjusted to 1 × 10⁴/ml, and poured by aliquot of 100 ml in a 96-well plate.
The 96-well plate added with fibroblasts was cultured for 24 hours, and samples serially diluted with PBS (-) were added by an amount of 10 µl. 48 hours after adding a test sample, 200 µl of PBS (-) was added to the 96-well plate, and approximately 1 min after, all the solution was removed. Immediately, 160 µl of DMSO was added to the plate. However, to the plates in which PtCl₄/HAP and H₂PtCl₆/HAP were added, DMSO was added in an amount of 200 µl, respectively. Each plate was shaken for 5 min in a shaker, to dissolve completely the formed formazan. As it was believed that precipitation of PtCl₄/HAP and H₂PtCl₆/HAP would suppress the colorization, the plates of PtCl₄/HAP and H₂PtCl₆/HAP were rested for 5 min, and 160 µl of the supernatant was moved to another 96-well plate. Test samples for each cell and each concentration, the test was performed for 4 wells or more, and the optical density was measured by using a microplate at 550 nm.
IC 50 and platinum content wherein IC50 is converted to platinum concentration by a toxicity test of PtCl₄/HAP and H₂PtCl₆/HAP during proliferation stage are shown in Table 5.

**[Table 5]**

| IC50 (ppm) in the toxicity test on platinum tetrachloride/hydroxyapatite (PtCl₄/HAP), and hexachloroplatinate/hydroxyapatite (H₂PtCl₆/HAP) during proliferation stage, and platinum content (ppm) wherein IC50 is converted to platinum concentration | | |
|---|---|---|
| | IC50 | Net Pt |
| PtCl₄/HAP | 29.8 | 3.0 |
| H₂PtCl₆/HAP | 25.2 | 2.5 |
| PtCl₄ | 4.0 | 2.3 |
| CDDP | 0.5 | 0.3 |

As a result of toxicity test during proliferation stage, IC50 of CDDP was 0.5 ppm, showing a potent toxicity. IC50 of PtCl₄/HAP was 29.8 ppm, and IC50 of H₂PtCl₆/HAP was 25.2 ppm, both being high values, compared to 4.0 ppm for PtCl₄ showing there is no potent suppressing effect. Moreover, when comparing these values by converting to platinum content, PtCl₄/HAP value was a significantly higher value compared to that of PtCl₄ or CDDP. Further, H₂PtCl₆/HAP value was also significantly higher compared to that of PtCl₄ or CDDP, showing that there was no potent suppressing effect.

### [Example 7]

### Toxicity test of platinum tetrachloride/hydroxyapatite (PtCl₄/HAP) and hexachloroplatinate/hydroxyapatite (H₂PtCl₆/HAP) during resting stage

Rat fibroblasts during proliferating stage were inoculated 2 times more than usual to a 96-well plate, that 100 µl of cell suspension at 2 × 10⁴ cells/ml. Subsequently, it was cultured for 72 hours, in order to make cells in a confluent condition. After confirming with an inverted microscope that cells were in a complete confluent condition in each well, medium of each well was changed. Then, serially diluted test samples were added to the wells by aliquot of 10 µl, by the above-mentioned method. Culture was continued 48 hours after the addition, and subsequently, MTT assay was performed for PtCl₄/HAP (particle diameter 0.1 µm or less), and H₂PtCl₆/HAP (particle diameter 0. 1 µm or less), with similar procedures to those described in the above "toxicity test of PtCl₄/HAP and H₂PtCl₆/HAP during proliferation stage".
IC 50 and platinum content wherein IC50 is converted to platinum concentration in a toxicity test of PtCl₄/HAP and H₂PtCl₆/HAP during resting stage are shown in Table 6.

**[Table 6]**

| IC50(ppm) in the toxicity test of platinum tetrachloride/hydroxyapatite (PtCl₄/HAP), and hexachloroplatinate/hydroxyapatite (H₂PtCl₆/HAP) during resting stage, and platinum content (ppm) wherein IC50 is converted to platinum concentration | | |
|---|---|---|
| | IC50 | Net Pt |
| PtCl₄/HAP | 202.6 | 20.3 |
| H₂PtCl₆/HAP | 160.0 | 16.0 |
| PtCl₄ | 23.8 | 13.8 |
| CDDP | 2.9 | 1.9 |

As cells at the resting stage scarcely undergo cell division, there is almost no possibility that platinum penetrates into cells, shows any effect on DNA, and inhibits its division, and the results show cell toxicity, that is an effect to kill cells, of platinum or drug itself. In this case, CDDP showed a higher cell-killing effect compared to the 2 other test samples, and net platinum content was 1.9 ppm.
In this toxicity test result, IC 50 of CDDP was 2.9 ppm, that is 1.9 ppm when converted to platinum content, showing a significant potent toxicity effect. However, IC50 of PtCl₄/HAP was 202.6 ppm, that is 20.3 ppm when converted to platinum content; IC50 of H₂PtCl₆/HAP was 160.0 ppm, that is 16.0 ppm when converted to platinum content; which values are significantly higher compared to that of CDDP, showing that cell toxicity is very low.

### [Example 8]

### Microscopic observation of toxicity effect during resting stage

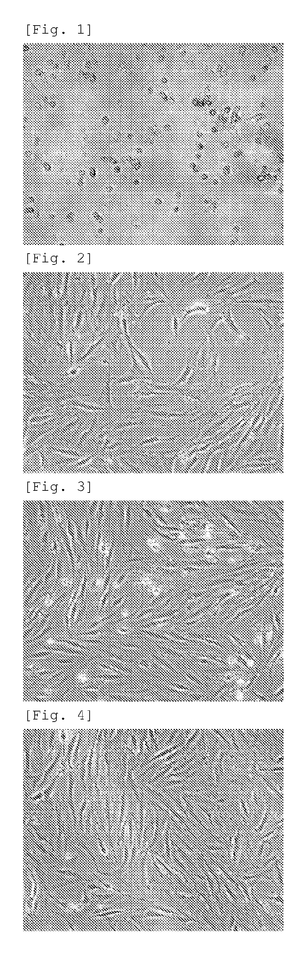

From the result of Example 7, 2 ppm which is a value of platinum content that kills 50% of fibroblasts in CDDP during resting stage was set as a standard. Then, CDDP, PtCl₄/HAP, PtCl₄ and HAP dispersion liquids in an amount corresponding to 2 ppm of platinum content, were added to the fibroblasts at confluency in a medium, and cultured for 48 hours. Microscopic pictures of the cultured cells are shown in Figs. 1 to 4.
Fig. 1 shows an image of cultured cells added with a CDDP dispersion liquid. Fig. 2 shows an image of cultured cells added with PtCl₄/HAP dispersion liquid. Fig. 3 shows an image of cultured cells added with PtCl₄ dispersion liquid. Fig. 4 shows an image of cultured cells added with a HAP dispersion liquid.

As a result, when PtCl₄/HAP was added, fibroblasts did not lose their original form, and almost all of the cells were located very closely each other by taking a spindle form, and showed a typical spindle form. When PtCl₄ was added, similarly as in the case of PtCl₄/HAP, most of fibroblasts showed a spindle form, while many cells wherein spindle form was deformed, or having a round form, were observed. When CDDP was added, almost no cell having a spindle form was observed, and most of the cells had a round form. When HAP dispersion liquid was added, cells showed a typical spindle form which is an original form of fibroblasts, and almost no abnormality was observed.
From the above result, it can be understood that vascular administration of an antitumor agent added with fine particles wherein hydroxyapatite is blended with platinum chloride or chloroplatinic acids, has a high antitumor effect, and it is an antitumor agent with less side-effect and low toxicity.

### Industrial Applicability

According to the present invention, an antitumor agent with less side-effect and showing an excellent anticancer effect, especially for lung cancer is provided.

## Claims

1. An antitumor composition wherein at least one or more of platinum chloride and/or chloroplatinic acids are supported by hydroxyapatite.

2. The antitumor composition according to claim 1, wherein platinum chloride and/or chloroplatinic acids is platinum tetrachloride, tetrachloroplatinate, hexachloroplatinate, disodium platinum tetrachloride, disodium platinum hexachloride, dipotassium platinum tetrachloride, or dipotassium platinum hexachloride.

3. The antitumor composition according to claim 1 or 2, wherein the maximum particle diameter of hydroxyapatite is 1 µm or less.

4. The antitumor composition according to claim 1 or 2, wherein the maximum particle diameter of hydroxyapatite is 0.1 µm or less.

5. The antitumor composition according to any one of claims 1 to 4, wherein hydroxyapatite supports platinum chloride and chloroplatinic acids in an amount of 0.1 to 100% to hydroxyapatite.

6. The antitumor composition according to any one of claims 1 to 5, wherein a mixture of an antitumor agent and hydroxyapatite is treated by grinding.

7. The antitumor composition according to any one of claims 1 to 6, wherein citrate and/or sodium citrate is used as a dispersant.
